## Europäisches Patentamt

## European Patent Office

## Office européen des brevets

(11) Numéro de publication: **0 174 245**
**B1**

## FASCICULE DE BREVET EUROPÉEN

(45) Date de publication du fascicule du brevet:
16.12.87

(51) Int. Cl.⁴: **C 07 K 5/08**, A 61 K 37/02

(21) Numéro de dépôt: **85401646.6**

(22) Date de dépôt: **14.08.85**

(54) **Nouveaux medicaments contenant des tripeptides.**

(30) Priorité: **16.08.84 FR 8412853**

(43) Date de publication de la demande:
**12.03.86 Bulletin 86/11**

(45) Mention de la délivrance du brevet:
**16.12.87 Bulletin 87/51**

(84) Etats contractants désignés:
**AT BE CH DE FR GB IT LI LU NL SE**

(56) Documents cité:
**FR-A-1 497 536**
**FR-A-2 268 792**

**JOURNAL OF THE CHEMICAL SOCIETY, Perkin Transactions I, Organic and Bio-organic Chemistry, 1975, pages 1424-1427, US; R.A.W. JOHNSTONE et al.: "Methods of peptide sequencing. Part II. Cyclisation of N-2-amino-6-nitrophenyl and N-3-amino-2-pyridyl derivatives of amino-acids and peptides"**
**UNLISTED DRUGS, vol. 32, no. 5, mai 1980, page 70, Spec. Libr. Associat., US; "FMLP"**

Le dossier contient des informations techniques présentées postérieurement au dépôt de la demande et ne figurant pas dans le présent fascicule.

(73) Titulaire: **RHONE- POULENC SANTE, Les Miroirs 18 Avenue d'Alsace, F-92400 Courbevoie Cédex (FR)**

(72) Inventeur: **Jolles, Pierre, 2, rue Jean- Francois Gerbillon, F-75270 Paris Cedex 06 (FR)**
Inventeur: **Migliore- Samour, Danièle, 64/66 avenue Charles- Gide, F-94270 Le Kremlin- Bicetre (FR)**
Inventeur: **Parker, Fabienne, 32, avenue Paul Painlevé, F-94200 Saint- Maur- Des- Fosses (FR)**

(74) Mandataire: **Pilard, Jacques, RHONE- POULENC RECHERCHES Service Brevets Pharma 25, Quai Paul Doumer, F-92408 Courbevoie Cedex (FR)**

## Description

La présente invention concerne des médicaments actifs comme immunostimulants contenant comme principe actif au moins un tripeptide constitué des aminoacides glycine, leucine et phénylalanine ou tyrosine.

Des tripeptides ou leurs dérivés, ainsi que leur procédé de préparation ont été décrits dans la littérature (FR 2 268 792, FR 1 497 597, J. Chem. Soc., Perkin Transaction J., 1975, pp. 1424-1427) sans que les propriétés physiologiques ou biologiques de ces produits aient été mises en évidence.

Dans le brevet européen EP 0 049 666 ont été décrites des substances biologiquement actives obtenues par fractionnement d'hydrolisats enzymatiques de la caséine humaine et les compositions qui les contiennent.

Plus particulièrement, le fractionnement, sur une colonne de Sephadex G-50 de la fraction hydrosoluble provenant du traitement de la caséine humaine, délipidée et solubilisée, par la trypsine pendant 24 heures à 37°C fournit trois fractions biologiquement actives désignées par "substances IV, V et VI" parmi lesquelles la "substance V", par purification sur une colonne DEAE - Sephadex A-25, fournit la substance neutre appelée "MJH 24", en éluant par une solution tampon à pH voisin de 8, puis les substances à caractère acide, appelées "MJH 63" et "MJH 65" en éluant par une solution tampon à pH voisin de 3,5.

Il a maintenant été trouvé que la purification de la substance "MJH 65" par filtration sur Sephadex G-15 puis par chromatographie liquide à haute performance (HPLC), après désalification, permet d'isoler un tripeptide dont la structure correspond à l'enchaînement:

Gly - Leu - Phe (I)

La structure du tripeptide de formule (I) a été déterminée:

- par hydrolyse totale par l'acide chlorhydrique 6N à 110°C pendant 18 heures
- par dansylation pour déterminer la nature de l'aminoacide N-terminal
- par action de la carboxypeptidase A
- par dégradation automatique avec un séquenceur BECKMANN, modèle 890 C.

Le tripeptide de formule (I) et les tripeptides analogues correspondant à la combinaison des trois aminoacides glycine, leucine, phénylalanine ou tyrosine, dont la synthèse peut être aisément réalisée par application des méthodes habituelles utilisées en chimie peptidique (par exemple selon le procédé décrit dans le brevet français FR 1 497 536) présentent des propriétés immunostimulantes remarquables.

Plus particulièrement, le tripeptide de formule (I) et les tripeptides analogues se sont montrés actifs à des concentrations compireses entre $10^{-5}$ et $10^{-7}$ M dans le test d'augmentation du nombre de macrophages plagocytaires selon la technique de J. Michl et Coll., J. exp. Med., 144, 1465 (1976).

La présente invention concerne les compositions pharmaceutiques qui contiennent au moins un tripeptide selon l'invention en association avec un ou plusieurs diluants ou excipients compatibles et pharmaceutiquement acceptables. Ces compositions peuvent être utilisées pour favoriser et augmenter l'élimination des agents infectieux. En tant qu'immunostimulants non spécifiques de l'immunité anti-infectieuse, les tripeptides selon l'invention sont administrés à des doses comprises entre 0,1 et 100 mg/kg par voie parentérale (intraveineuse, sous-cutanée, intramusculaire), intranasale, buccale ou rectale.

Comme compositions solides pour administration orale peuvent être utilisés des comprimés, des pilules, des poudres ou des granulés. Dans ces compositions, le produit actif est mélangé à un ou plusieurs diluants tels que saccharose, lactose ou amidon. Ces compositions peuvent également comprendre des substances autres que les diluants, par exemple un lubrifiant, tel que le stéarate de magnésium.

Comme compositions liquides pour administration orale, on peut utiliser des émulsions pharmaceutiquement acceptables, des solutions, des suspensions, des sirops ou des élixirs contenant des diluants inertes, tels que l'eau ou l'huile de paraffine. Ces compositions peuvent comprendre des substances autres que les diluants, par exemple des produits mouillants, édulcorants ou aromatisants.

Les compositions pour administration parentérale peuvent être des solutions stériles aqueuses, des suspensions ou des émulsions. Comme véhicule, on peut employer le polyéthylèneglycol, un polypropylèneglycol, des huiles végétales, en particulier l'huile d'olive, et les esters organiques injectables, par exemple l'oléate d'éthyle. Ces compositions peuvent contenir également des adjuvants, en particulier des agents mouillants, émulsifiants ou dispersants.

La stérilisation peut se faire de plusieurs façons, par exemple à l'aide d'un filtre bactériologique, en incorporant à la composition des agents stérilisants ou par chauffage. Elles peuvent être également préparées sous forme de compositions solides, rendues stériles par exemple par irradiation, qui peuvent être dissoutes dans de l'eau stérile ou dispersées dans tout autre milieu stérile injectable, éventuellement au moment de l'emploi.

Les compositions pour administration intranasale peuvent être des solutions stériles aqueuses, des suspensions ou des émulsions qui peuvent être éventuellement associées à un agent propulseur compatible.

Les compositions pour administration rectale sont des suppositoires qui peuvent contenir, outre le produit actif, des excipients tels que le beurre de cacao ou un glycéride semi-synthétique.

Les exemples suivants illustrent l'isolement du tripeptide de formule I et les compositions qui le contiennent.

## Exemple 1

La substance "MJH 65" dont l'isolement est décrit dans l'exemple 2 du brevet européen EP 0 049 666 est purifiée par filtration sur une colonne de Sephadex G-15. On utilise une colonne de 120 cm de hauteur et de 1,2 cm de diamètre. On élue avec de l'acide acétique à 10 % en recueillant des fractions de 0,6 cm$^3$ avec un débit de 9 cm$^3$/heure.

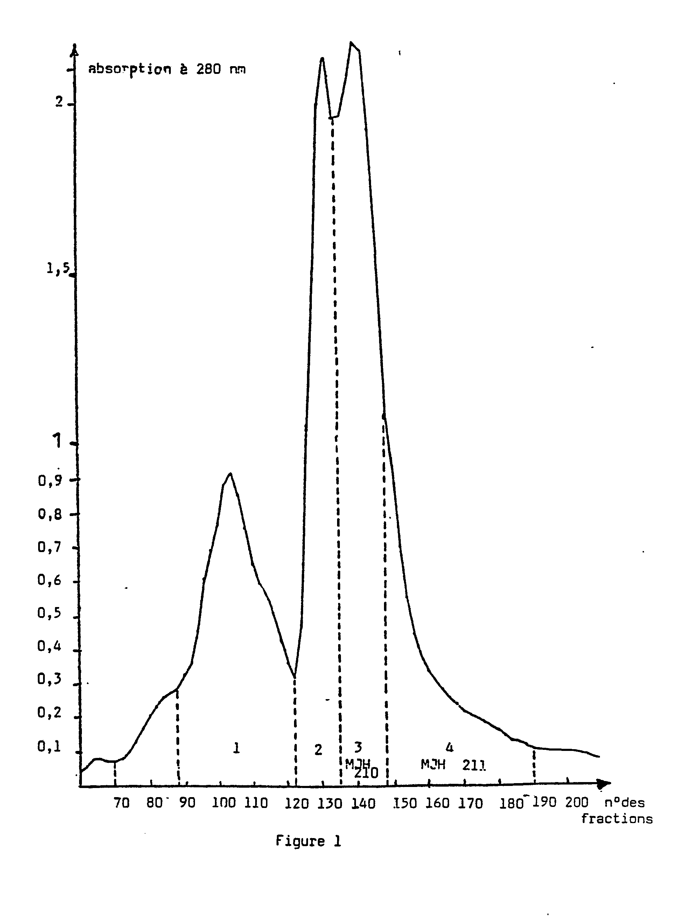

Le diagramme d'élution est représenté par la figure 1 dans laquelle sont portés, en abscisses, le numéro des fractions et, en ordonnées, l'absorption à 280 nm.

On recueille ainsi la fraction "MJH 210", éluée entre 81,6 et 88,8 cm$^3$ (fractions 136 à 148) et la fraction "MJH 211", éluée entre 88,9 et 114 cm$^3$ (fractions 149 à 190).

La fraction "MJH 210" est désalifiée sur une colonne de Dowex 50X4 de hauteur 14 cm et de diamètre 1,4 cm.

L'élution est effectuée en recueillant des fractions de 4,3 cm$^3$ avec un débit de 24 cm$^3$/heure en utilisant comme éluants 100 cm$^3$ d'acide chlorhydrique 0,05N, 100 cm$^3$ d'eau puis de l'ammoniaque 2M.

On recueille ainsi une fraction "3" éluée entre 34,4 et 81,7 cm$^3$ avec l'ammoniaque 2M.

Cette fraction "3" est purifiée par HPLC en phase "reverse" sur une colonne semi-préparative (colonne C$_{18}$ -μ Bondapack, Waters) dont la longueur est de 30 cm et dont le diamètre est de 7,8 mm. On recueille des fractions de 0,5 cm$^3$, la vitesse d'élution étant de 1 cm$^3$/minute.

Au départ, la colonne est tamponnée par de l'acide trifluoroacétique à 0,1 % (éluant A).

On prépare un éluant contenant de l'acide trifluoroacétique (0,1 % en volume) et de l'acétonitrile (70 % en volumes) dans l'eau (éluant B).

L'élution est effectuée en utilisant un gradient linéaire d'élution et en opérant selon le tableau suivant:

| Temps (minutes) | Eluant A | Eluant B |
|---|---|---|
| 0 | 100 | 0 |
| 10 | 100 | 0 |
| 110 | 50 | 50 |
| 140 | 0 | 100 |

L'élution est suivie par lecture à 280 nm et par la fluorescence après réaction à la fluorescamine à 490 nm.

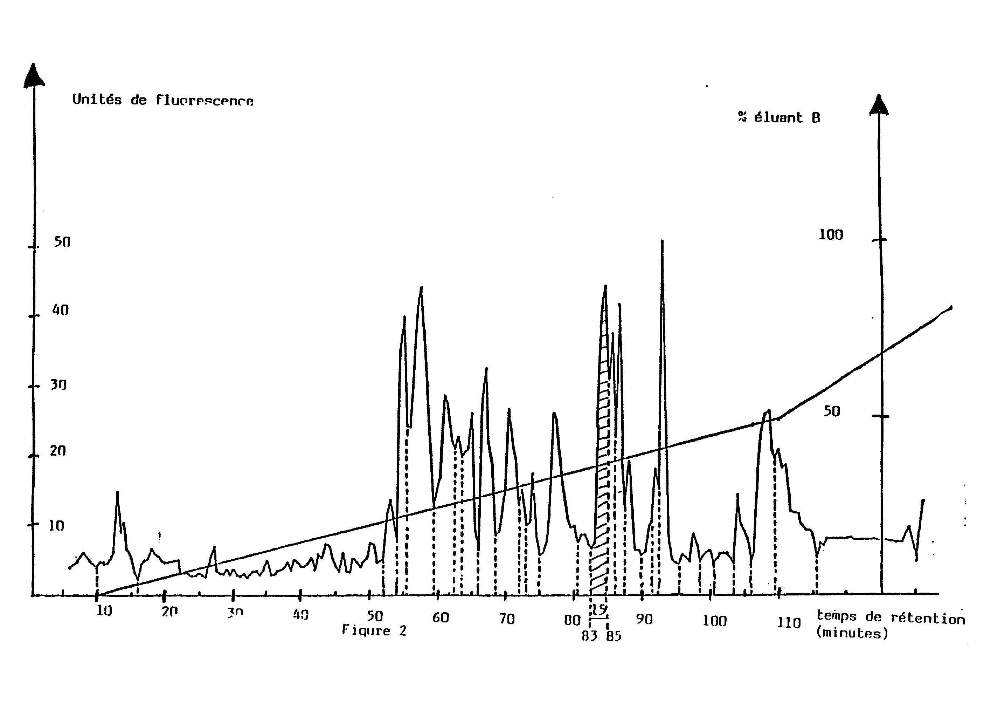

Le diagramme d'élution est représenté par la figure 2.

La fraction n° 15, éluée entre 83 et 85 minutes contient le tripeptide de formule (I) pur.

La structure du tripeptide de formule (I) est déterminée :
- par l'hydrolyse totale par l'acide chlorhydrique 6N à 110° C pendant 18 heures qui montre la présence de glycine (Gly) = 1, de leucine (Leu) = 1 et de phénylalanine (Phe) = 1.
- par dansylation afin de déterminer la nature de l'aminoacide N-terminal, qui est la glycine.
- par analyse par le séquenceur Beckmann, modèle 890C, programme Quadrol 0,1M avec détermination des différentes étapes grâce à la caractérisation des phénylthiohydantoine-acides aminés (détermination par HPLC et révélation sur plaques).

## Exemple 2

Par applications des méthodes habituelles utilisées en chimie peptidique, les tripeptides suivants sont préparés :
- Gly - Phe - Leu
- Leu - Gly - Phe
- Phe - Leu - Gly
- Leu - Phe - Gly
- Phe - Gly - Leu
- Gly - Tyr - Leu
- Leu - Gly - Tyr
- Tyr - Leu - Gly
- Leu - Tyr - Gly
- Tyr - Gly - Leu
- Gly - Leu - Tyr

## Exemple 3

On prépare selon la technique habituelle une composition liquide administrable par voie intraveineuse ayant la composition suivante :
- tripeptide de formule (I) 50 mg
- soluté injectable 5 cm$^3$.

**Revendications** pour les etats contractants BE, CH, DE, FR, GB, IT, LI, LU, NL, SE.

1. Médicament actif comme immunostimulant caractérisé en ce qu'il est constitué par un tripeptide constitué des aminoacides glycine, leucine et phénylalanine ou tyrosine.

2. Médicament nouveau caractérisé en ce que le tripeptide est Gly - Leu - Phe.

3. Composition pharmaceutique caractérisée en ce qu'elle contient au moins un médicament selon la revendication 1 en association avec un ou plusieurs diluants ou adjuvants compatibles et pharmaceutiquement acceptables.

4. Composition pharmaceutique caractérisée en ce qu'elle contient un médicament selon la revendication 2 en association avec un ou plusieurs diluants compatibles et pharmaceutiquement acceptables.

**Revendications** pour l'etat contractant:
AT

1. Procédé de préparation d'une composition pharmaceutique immunostimulante caractérisée en ce que l'on associe un tripeptide constitué des aminoacides glycine, leucine et phénylalanine ou tyrosine avec un ou plusieurs diluants compatibles et pharmaceutiquement acceptables.

2. Procédé selon la revendication 1 caractérisé en ce que le tripeptide est Gly-Leu-Phe.

**Patentansprüche** für die Vertragsstaaten BE, CH, DE, FR, GB, IT, LI, LU, NL, SE.

1. Als Immunostimulans wirksames Arzneimittel, dadurch gekennzeichnet, daß es aus einem Tripeptid besteht, das aus den Aminosäuren Glycin, Leucin und Phenylalanin oder Tyrosin besteht.

2. Arzneimittel gemäß Anspruch 1, dadurch gekennzeichnet, daß das Tripeptid Gly - Leu - Phe ist.

3. Pharmazeutische Zusammensetzung, dadurch gekennzeichnet, daß sie wenigstens ein Arzneimittel gemäß Anspruch 1 in Assoziation mit einem oder mehreren verträglichen und pharmazeutisch annehmbaren Verdünnungs- oder Hilfsmitteln enthält.

4. Pharmazeutische Zusammensetzung, dadurch gekennzeichnet, daß sie ein Arzneimittel gemäß Anspruch 2 in Assoziation mit einem oder mehreren verträglichen und pharmazeutisch annnehmbaren Verdünnungsmitteln enthält.

**Patentansprüche** für den Vertragsstaat AT:

1. Verfahren zur Herstellung eines immunostimulierenden pharmazeutischen Präparats, dadurch gekennzeichnet, daß man ein Tripeptid, bestehend aus den Aminosäuren Glycin, Leucin und Phenylalanin oder Tyrosin, mit einem oder mehreren verträglichen und pharmazeutisch zulässigen Verdünnungsmitteln vereinigt.

2. Verfahren nach Anspruch 1, dadurch gekennzeichnet, daß das Tripeptid Gly-Leu-Phe ist.

**Claims** for the contracting states: BE, CH, DE, FR, GB, IT, LI, LU, NL, SE.

1. Medicinal product which is active as an immunostimulant, characterized in that it consists of a tripeptide consisting of the amino acids glycine, leucine and phenylalanine or tyrosine.

2. Medicinal product according to Claim 1, characterized in that the tripeptide is Gly-Leu-Phe.

3. Pharmaceutical composition, characterized in that it contains at least one medicinal product according to Claim 1 in combination with one or more diluents or additives which are compatible and pharmaceutically acceptable.

4. Pharmaceutical composition, characterized in that it contains a medicinal product according to Claim 2 in combination with one or more diluents which are compatible and pharmaceutically acceptable.

**Claims** for the contracting state: AT

1. Process for preparing an immunostimulatory pharmaceutical composition, characterized in that a tripeptide consisting of the amino acids glycine, leucine and phenylalanine or tyrosine is combined with one or more diluents which are compatible and pharmaceutically acceptable.

2. Process according to Claim 1, characterized in that the tripeptide is Gly-Leu-Phe.

Figure 1

Figure 2